# EUROPÄISCHE PATENTSCHRIFT

(11) **EP 0 570 356 B1**
(45) Veröffentlichungstag und Bekanntmachung des Hinweises auf die Patenterteilung: **17.02.1999**
(21) Anmeldenummer: 93890099.0
(22) Anmeldetag: 12.05.1993
(51) Int. Cl.: G01N 33/86

(54) **Reagens zur Bestimmung der aktivierten partiellen Thromboplastinzeit (aPTT)**
Reagent for the determination of the activated partial thromboplastin time (aPII)
Réactif pour la détermination du temps de thromboplastinee partiellement active (IIPa)

(30) Priorität: 15.05.1992 AT 998/92; 30.04.1993 AT 841/93
(43) Veröffentlichungstag der Anmeldung: 18.11.1993
(73) Patentinhaber: IMMUNO Aktiengesellschaft, A-1221 Wien (AT)
(72) Erfinder: Lang, Hartmut, Dr., A-1230 Wien (AT); Moritz, Berta, Dr., A-1030 Wien (AT)
(74) Vertreter: Weinzinger, Arnulf, Dipl.-Ing.

(56) Entgegenhaltungen:
- EP-A- 0 107 383
- EP-A- 0 123 883
- WO-A-91/16453
- WO-A-92/08479
- CH-A- 678 892
- BIOCHEMISTRY. Bd. 19, Nr. 7, Juli 1980, EASTON, PA US Seiten 1322 - 1330 KAZUO FUJIKAWA, RONALD L. HEIMARK, KOTOKU KURACHI, AND EARL W. DAVIE 'Activation of Bovine Factor XII (Hageman Factor) by Plasma Kallikrein'
- BLOOD Bd. 59, Nr. 1, Januar 1982, NEW YORK NY US Seiten 69 - 75 GUIDO TANS AND JOHN H. GRIFFIN 'Properties of Sulfatides in Factor-XII-Dependent Contact Activation'
- LOTHAR THOMAS 'LABOR UND DIAGNOSE' 1988 , DIE MEDIZINISCHE VERLAGSGESELLSCHAFT , MARBURG DE

## Beschreibung

Die Erfindung betrifft einen Initiator der intrinsischen Gerinnung, ein Reagens zur Bestimmung der aktivierten partiellen Thromboplastinzeit (aPTT) sowie ein Verfahren zur Herstellung dieses Reagens bzw. zur Bestimmung der aPTT.

Die aktivierte partielle Thromboplastinzeit einer Plasmaprobe dient als "screening"-Test zur globalen Erfassung der Aktivität von Gerinnungsfaktoren der intrinsischen Gerinnung. Störungen im Gerinnungsablauf bedingen Gerinnungszeiten, die außerhalb des Normalbereiches liegen. Die Verlängerung der aPTT, wie sie bei der Hämophilie A auftritt, manifestiert z.B. einen Mangel an Gerinnungsfaktor VIII. Zusätzlich wird der aPTT-Test häufig zur Überwachung einer Heparintherapie eingesetzt.

Ein Reagens zur Bestimmung der aPTT enthält einen Aktivator, um die Kaskade der intrinsischen Gerinnung bis zur Bildung von aktiviertem Faktor IX zu initiieren. Des weiteren werden zur Aktivierung von Faktor X und Prothrombin Phospholipide benötigt, weshalb diese ebenfalls in einem aPTT-Reagens enthalten sind. Das Reagens wird mit einer Plasmaprobe in Kontakt gebracht, worauf nach einer definierten, möglichst kurzen Aktivierungszeit Calcium zugesetzt wird und die Zeit bis zur Bildung eines Fibrinclots gemessen wird.

An das Testsystem der aPTT werden eine Vielzahl von Anforderungen gestellt. Für Routineuntersuchungen ist die einfache Handhabung eines aPTT-Reagens unbedingte Voraussetzung. Ebenso muß die Stabilität der Inhaltsstoffe sehr hoch sein, um eine gleichbleibende Gerinnungszeit über einen langen Zeitraum hinweg zu gewährleisten. Dies ermöglicht auch den kostengünstigen Einsatz des Reagens. Auch ist für die aPTT als Globaltest eine ausgewogene Sensitivität gegenüber allen zu erfassenden Parametern erforderlich.

Übliche Reagenzien zur Bestimmung der aPTT enthalten als Initiator der intrinsischen Gerinnung einen oberflächenaktiven Feststoff. Durch die Verwendung einer oberflächenaktiven Matrix wird zuerst Faktor XII aktiviert, vergleichbar mit den physiologischen Bedingungen. Als Feststoffaktivator werden etwa Kaolin, Celit und Glas eingesetzt. Kaolin ist beispielsweise in handelsüblichen Reagenzien zur Bestimmung der aPTT als Suspension enthalten (Pathromtin®, Fa. Behringwerke (1990) oder PTT Reagens, Fa. Boehringer Mannheim (1986)). Als Phospholipid wird ein lyophilisierter Lipidextrakt aus Humanplazenten bzw. lyophilisiertes Kephalin eingesetzt. Die Phospholipide werden jedoch erst vor der Durchführung der Bestimmung mit Kaolin zu einem gebrauchsfertigen Reagens gemischt. Die getrennte Lagerung von Kaolin und Phospholipiden ist vor allem aus Stabilitätsgründen erforderlich.

Bei der Lyophilisierung von Lipiden mit Kaolin würden Lipide großteils inaktiviert werden. So tritt etwa bei der Herstellung von PTT-IMMUNO (Fa. Immuno) bei der gemeinsamen Lyophilisierung von Kaolin und Phospholipiden (extrahiert aus Schweinehirn) ein Verlust von bis zu 50 % der Phospholipidaktivität auf.

Als Alternative zu Feststoffaktivatoren können auch flüssige Aktivatoren, wie Ellagsäure und ihre Derivate, oder gelöste Sulfatide eingesetzt werden. Die Empfindlichkeit eines Flüssigaktivatoren enthaltenden Tests gegenüber Heparin ist jedoch verschlechtert, weshalb diverse Zusätze zur Empfindlichkeitsverbesserung vorgeschlagen werden. Gemäß der WO 91/16453 enthält ein heparinempfindliches Reagens auf Basis von Ellagsäure zusätzlich Dextransulfat.

Ein Nachteil von Kaolin bzw. von Feststoffaktivatoren im allgemeinen und Ellagsäure bzw. -derivaten ist die lange Aktivierungszeit der ersten Phase der intrinsischen Gerinnung, wodurch eine relativ lange Inkubationszeit von mindestens 4 min zur Aktivierung notwendig ist. Ein weiterer Nachteil besteht in der relativ langen Gerinnungszeit in Gegenwart von Kaolin. Eine höhere Kaolinkonzentration im Reagens kann jedoch die Aktivierungszeit verkürzen. Kaolin, das sich aus der Suspension absetzt, kann aber zu einer verlängerten Gerinnungszeit führen bzw. Fehlergebnisse bei der Ermittlung des Gerinnungsendpunktes mittels gebräuchlicher Koagulometer hervorrufen. Es muß daher getrachtet werden, die Konzentrationen an Feststoffaktivator möglichst gering zu halten.

Die Erfindung stellt sich die Aufgabe, ein standardisiertes Reagens mit hoher Stabilität zur Bestimmung der aPTT zur Verfügung zu stellen, welches einfach zu handhaben und kostengünstig ist und sowohl kurze Aktivierungszeiten als auch eine hohe Empfindlichkeit gegenüber Einzelfaktoren der Gerinnungskaskade mit sich bringt.

Die Lösung der gestellten Aufgabe wird erfindungsgemäß durch einen Initiator der intrinsischen Gerinnung von Blut, Plasma oder Blutderivaten ermöglicht, der ein Gemisch aus Sulfatiden und einem Feststoffaktivator, vorzugsweise Kaolin, enthält.

Das erfindungsgemäße Reagens enthält als Initiator der intrinsischen Gerinnung ein Gemisch aus Sulfatiden (z.B. von der Fa. Pentapharm) und einen Feststoffaktivator. Weiters sind Phospholipide enthalten. Die nur kurze Aktivierungszeit, welche bei Verwendung von Sulfatiden zur Aktivierung von Faktor XII benötigt wird, ist durch die gemeinsame Verwendung mit Kaolin kaum verlängert. Mit der Verwendung des erfindungsgemäßen Reagens wird somit eine für Routineuntersuchungen praktikable Vorgangsweise der kurzen Inkubationszeit ermöglicht. Gleichzeitig wird aber auch die Heparinempfindlichkeit durch den Einsatz des Feststoffaktivators verbessert.

Die Verwendung des erfindungsgemäßen Initiators in einem aPTT Reagens ermöglicht also überraschenderweise die Kombination der Vorteile beider Einzelaktivatoren, jedoch nicht deren Nachteile.

Dies war nicht vorherzusehen. Eher war zu vermuten, daß die Kombination der Sulfatide mit Kaolin als Initiator der intrinsischen Gerinnung im Reagens zu einer Verminderung der Aktivität führt. Gemäß dem Vorurteil der Fachwelt, daß Schwierigkeiten bei der Kombination von Aktivatoren mit unterschiedlichen Reaktionsmechanismen entstehen würden, war es überraschend, daß das erfindungsgemäße Reagens sehr gut standardisierbar ist. Dies ist Voraussetzung für eine hohe Reproduzierbarkeit des Bestimmungsverfahrens.

Im erfindungsgemäßen Reagens wird die Menge an Kaolin gering gehalten. Das vermehrte Absetzen von Kaolin wird verhindert, Schwierigkeiten beim Betrieb von Detektionsgeräten werden vermieden.

Der erfindungsgemäße Initiator kann ohne Schwierigkeiten gemeinsam mit Phospholipiden zu einem kostengünstigen Reagens lyophilisiert werden. Eine mögliche negative Wechselwirkung der Einzelkomponenten ist ohne Bedeutung.

Die Erfindung betrifft daher ein Reagens zur Bestimmung der aPTT von Blut, Plasma oder Blutderivaten, das dadurch gekennzeichnet ist, daß es ein Gemisch aus Sulfatiden und einem Feststoff-Aktivator, vorzugsweise Kaolin, als Initiator der intrinsischen Gerinnung und weiters Phospholipide enthält.

Die Konzentrationen an Kaolin bzw. Sulfatiden im Reagens betragen für Kaolin zwischen 0,1 und 0,6 Gew.%, vorzugsweise 0,1 bis 0,3 Gew.%, und für Sulfatide 0,001 bis 0,03 Gew.%, vorzugsweise 0,002 bis 0,01 Gew.%.

Das Reagens enthält vorzugsweise chemisch reine Phospholipide in einer standardisierten Mischung. Es hat sich gezeigt, daß dadurch eine unerwartet hohe Reproduzierbarkeit des Testverfahrens erreicht wrid. Die Mischung der Phospholipide enthält beispielsweise Phosphatidylserin, Phosphatidylcholin und Cholesterin.

In der Mischung befinden sich vorzugsweise 5 bis 50 % Phosphatidylserin, 15 bis 60 % Phosphatidylcholin, 15 bis 60 % Phosphatidylethanolamin, 0 bis 20 % Cholesterin und 5 bis 20 % Phosphatidinsäure, bezogen auf den Gesamtphospholipidgehalt. Als standardisierte Mischung wird z.B. 0,2 mg Phosphatidylserin, 0,85 mg Phosphatidylcholin, 0,65 mg Phosphatidylethanolamin, 0,15 mg Cholesterin und 0,15 mg Phosphatidinsäure pro ml eventuell verdünnt eingesetzt.

Die Phospholipide sind vorteilhafterweise in einer Weise gereinigt, sodaß unerwünschte Fällungsreaktionen, die in Gegenwart von Polybren auftreten können, vermieden werden.

Eine bevorzugte Ausführungsform des Reagens ist dadurch gekennzeichnet, daß es weiters Oxidationshemmer und Empfindlichkeitsverbesserer enthält. Als Oxidationshemmer zur Erhöhung der Lagerstabilität des Reagens im lyophilisierten oder flüssigen Zustand werden beispielsweise Cystein, Harnsäure, Vitamin C oder Vitamin E eingesetzt. Zusätze, wie Alkalisalze, eventuell gemeinsam mit Aminosäuren, erhöhen die Sensitivität des Tests gegenüber Einzelfaktoren. Es hat sich gezeigt, daß sich ein Reagens enthaltend Cäsiumchlorid (5 bis 25 mg/ml) und Arginin (2,5 bis 20 mg/ml), vorzugsweise 16,8 mg CsCl/ml und 10,5 mg Arginin/ml, nach entsprechender Verdünnung besonders gut zur Bestimmung der aPTT einer Plasmaprobe mit einem Mangel an Einzelfaktoren (Faktor VIII) eignet.

Eine weitere bevorzugte Ausführungsform des Reagens ist dadurch gekennzeichnet, daß es weiters ein chromogenes Substrat enthält, mit dem gegebenenfalls die Gerinnungszeit photometrisch durch Farbentwicklung bestimmbar ist. Die Erfassung des Endproduktes ist aber auch durch die photometrische Bestimmung der Veränderung der Lichtdurchlässigkeit möglich.

Vorteilhafterweise enthält das Reagens weiters Stabilisatoren, vorzugsweise Albumin und/oder Puffersubstanzen.

Das Verfahren zur Herstellung dieses Reagens ist einfach. Sulfatide werden in einer wässerigen Suspension des Feststoffaktivators mit Phospholipiden und gegebenenfalls mit weiteren Zusätzen vermischt und gegebenenfalls lyophilisiert.

Die Herstellung des Reagens kann aber auch dadurch erfolgen, daß Sulfatide, der Feststoffaktivator und Phospolipide, gegebenenfalls nach Lyophilisierung, gegebenenfalls mit weiteren Zusätzen im festen Zustand vermischt werden.

Das erfindungsgemäße Reagens eignet sich sowohl zur Überwachung einer Heparintherapie als auch zur Bestimmung von Einzelfaktoren, also Gerinnungsfaktoren bzw. Inhibitoren der intrinsischen Gerinnung.

Die Erfindung betrifft weiters ein Verfahren zur Bestimmung der partiellen Thromboplastinzeit (aPTT) von Proben von Blut, Plasma oder Blutderivaten, welches dadurch gekennzeichnet ist, daß die Probe mit einem erfindungsgemäßen Reagens in Kontakt gebracht wird, nach einer vorbestimmten Aktivierungszeit Calciumionen zugesetzt werden und die Gerinnungszeit bestimmt wird.

Durch den Zusatz von heparinneutralisierenden Substanzen kann ein Gerinnungsfaktor-Mangel auch in heparinisierten Plasmaproben bestimmt werden. Polybren neutralisiert beispielsweise vorhandenes Heparin und macht damit das erfindungsgemäße Reagens gegenüber einem Heparingehalt der Probe unempfindlich.

Eine weitere bevorzugte Ausführungsform des erfindungsgemäßen Verfahrens ist also dadurch gekennzeichnet, daß die Probe vor Zugabe des erfindungsgemäßen Reagens mit einer Heparin-neutralisierenden Substanz, wie Polybren, Protaminsulfat, usw. versetzt wird.

Weiters betrifft die Erfindung die Verwendung des erfindungsgemäßen Verfahrens zur Bestimmung der Aktivität von Faktoren der intrinsischen Gerinnung und deren Inhibitoren, wobei eine Plasmaprobe mit einem erfindungsgemäßen Reagens in Kontakt gebracht wird, nach einer vorbestimmten Aktivierungszeit Calciumionen zugesetzt werden und die Gerinnungszeit bestimmt wird, worauf das erhaltene Ergebnis mit dem einer Standardprobe mit bekannter Aktivität der Faktoren in Bezug gesetzt wird.

Es wurde weiters gefunden, daß die Bestimmung der aPTT von gegebenenfalls Heparin- oder Heparinoid-hältigen(m) Blut, Plasma oder Blutderivaten auch ohne Verwendung eines Gemisches aus Sulfatiden und Feststoffaktivator durchgeführt werden kann, wenn in einem Reagens zur Bestimmung der aPTT neben einem Initiator der intrinsischen Gerinnung, sei es nun ein Feststoffaktivator oder ein Flüssigaktivator oder eine Kombination derselben, ein Heparin-neutralisierendes Agens und Phospholipide enthalten sind.

Als Heparin-neutralisierende Agentien kommen insbesondere Polybren, Protamin (Protaminsalze) oder Heparinase im erfindungsgemäßen Reagens zur Anwendung.

Die Konzentrationen der zur Anwendung kommenden Heparin-neutralisierenden Agentien richtet sich größenordnungsmäßig nach der Heparin-Konzentration in der zu untersuchenden Probe, und liegt üblicherweise, etwa im Falle von Polybren, im Bereich zwischen 1 und 500 mg/l. In standardmäßigen Verfahren werden zwischen 2 und 100 mg/l Polybren verwendet.

Der Vorteil eines Reagens enthaltend ein Heparin-neutralisierendes Agens besteht insbesondere in der Tatsache, daß die Bestimmung der aPTT und der Einzel faktoren in einer Blut- oder Plasmaprobe unabhängig davon erfolgen kann, ob die Probe Heparin oder Heparinoide enthält oder nicht.

Die Erfindung betrifft weiters ein Set bestehend aus a) einem Reagens zur Bestimmung der aPTT, enthaltend einen Initiator der intrinsischen Gerinnung und Phospholipide, und b) einem Heparin-neutralisierenden Agens.

Obwohl die aPTT oft zur Überwachung einer Heparintherapie eingesetzt wird, wobei eine Heparin-Sensitivität erwünscht ist, gibt es doch viele Fälle, bei denen das intakte intrinsische System oder Faktoren des intrinsischen Systems unabhängig vom Heparingehalt untersucht werden müssen.

Sollte nämlich Heparin in der Probe enthalten sein und dadurch die aPTT bei Messung mit zum Stand der Technik gehörenden Reagentien verzögert werden, wird ein zu geringer Anteil an Gerinnungsfaktoren vorgetäuscht.

Die Erfindung wird durch die nachfolgenden Beispiele noch näher erläutert.

### 1. Vergleich der aPTT bei Verwendung von diversen Reagenzien

Die untersuchten Reagenzien zur Bestimmung der aPTT enthielten als Initiator der intrinsischen Gerinnung Sulfatide (Fa. Pentapharm) und Kaolin in unterschiedlichen Mischungsverhältnissen in 200 mM Hepes-Glycin-Puffer (pH 6,8). Weiters war eine Mischung aus 4,8 µg Phosphatidylserin, 20,7 µg Phosphatidylcholin, 15,9 µg Phosphatidyläthanolamin, 3,7 µg Cholesterin und 3,7 µg Phosphatidinsäure pro ml im Reagens vorhanden.

0,1 ml Normalplasma (NP) (Reference Plasma 100 %, Fa. Immuno) bzw. ein abnormales Plasma (AP) (Immunocontrol Plasma Abnormal, Fa. Immuno) wurden mit 0,1 ml Reagens versetzt. Nach einer 2-minütigen Inkubationszeit bei 37°C wurden 0,1 ml 0,025 M CaCl₂-Lösung zugesetzt und die Zeit bis zum Gerinnungspunkt mit einem Schnitger-Gross-Koagulometer (Fa. Amelung) gemessen.

Aus der Tabelle 1 ist ersichtlich, daß durch die Gegenwart von Sulfatiden im Reagens die Gerinnungszeit vom Normalplasma (NP) verkürzt ist. Kaolin im Reagens verbessert aber auch die Empfindlichkeit, ausgedrückt durch das Verhältnis der Gerinnungszeiten von AP und NP (siehe Tabelle 2).

**Tabelle 1:**

| aPTT (s) | | | | |
|---|---|---|---|---|
| | Kaolin (%) | 0 | 0,15 | 0,30 |
| | Sulfatid (%) | | | |
| NP | 0 | X | 78,1 | 70,1 |
| NP | 0,005 | 34,4 | 38,4 | 38,6 |
| NP | 0,01 | 39,1 | 41,1 | 41,8 |

**Tabelle 2:**

| Verhältnis der aPTT (AP/NP) | | | |
|---|---|---|---|
| Kaolin (%) | 0 | 0,15 | 0,30 |
| Sulfatid (%) | | | |
| 0 | X | 2,20 | 2,12 |
| 0,005 | 2,00 | 2,11 | 2,21 |
| 0,01 | 2,01 | 2,15 | 2,23 |

### 2. Heparinempfindlichkeit

Unter Verwendung der Reagenzien aus 1. wurden Normalplasma (siehe 1.) und heparinisiertes Normalplasma (0,37 E Heparin/ml) untersucht. Das Verhältnis der aPTT von heparinisiertem Normalplasma zu Normalplasma ist für die verschiedenen Reagenzien in Tabelle 2 angegeben. Die Empfindlichkeit gegenüber Heparin wird durch einen höheren Kaolingehalt im Reagens verbessert.

**Tabelle 3:**

| | Verhältnis der aPTT (+/- Heparin) | | |
|---|---|---|---|
| Kaolin (%) | 0 | 0,15 | 0,30 |
| Sulfatide (%) | | | |
| 0 | X | 1,50 | 1,66 |
| 0,005 | 1,18 | 1,28 | 1,37 |
| 0,01 | 1,23 | 1,31 | 1,41 |

### 3. Empfindlichkeit gegenüber Faktor VIII

Unter Verwendung der Reagenzien aus 1. wurden Normalplasma (siehe 1.) und Control Plasma (Fa. Immuno; enthielt < 30 % Faktor VIII bezogen auf den Faktor VIII-Gehalt von Normalplasma (= 100 %)) untersucht. Das Verhältnis der aPTT des Control Plasmas zu Normalplasma ist für die verschiedenen Reagenzien in Tabelle 4 angegeben. Die Empfindlichkeit gegenüber Faktor VIII wird durch den Gehalt an Sulfatiden im Kaolin-hältigen Reagens verbessert.

**Tabelle 4:**

| | Verhältnis der aPTT Control Plasma (< 30 % Faktor VIII/Normalplasma) | | |
|---|---|---|---|
| Kaolin (%) | 0 | 0,15 | 0,30 |
| Sulfatide (%) | | | |
| 0 | X | 1,48 | 1,51 |
| 0,005 | 1,66 | 1,75 | 1,74 |
| 0,01 | 1,71 | 1,72 | 1,70 |

### 4. Faktor VIII-Bezugskurve

Zur Erstellung einer Bezugskurve für Faktor VIII wurde Reference Plasma 100 % (Fa. Immuno) 1 : 5 mit Imidazolpuffer (3,4 g/l Imidazol, 5,85 g/l NaCl, pH 7,4) verdünnt. 0,1 ml dieser Verdünnung wurden mit 0,1 ml Phospholipid-Kaolin-Sulphatid-Reagens (Zusammensetzung der Phospholipide wie in Beispiel 1; 0,3 % Kaolin und 0,01 % Sulfatide in 200 mM Hepes-Glycin-Puffer, pH 6,8) vermischt und 4 min bei 37°C inkubiert. Gleichzeitig mit der Zugabe von 0,1 ml Calciumchlorid (25 mM) wurde die Stoppuhr in Gang gesetzt und die Zeit bis zum Gerinnungsendpunkt mittels eines Schnitger-Gross-Koagulometers (Fa. Amelung) bestimmt.

Das 1 : 5 vorverdünnte Plasma entspricht 100 % Faktor VIII. Weitere Verdünnungen wurden gemäß einer geometrischen Verdünnungsreihe (1 : 1 = 100 % bis 1 : 128 = 0,78 %) getestet. In Tabelle 5 sind die Meßergebnisse angeführt.

**Tabelle 5:**

| Faktor VIII (%) | Gerinnungszeit (s) |
|---|---|
| 100 | 78,5 |
| 50 | 91,0 |
| 25 | 101,7 |
| 12,5 | 115,3 |
| 6,25 | 128,9 |
| 3,13 | 139,9 |
| 1,56 | 148,6 |
| 0,78 | 159,4 |

### 5. aPTT Bestimmung mit PTT Reagentien unterschiedlicher Zusammensetzung mit und ohne Zusatz von Polybren.

Fünf verschiedene Reagentien werden in den vorliegenden Beispielen sowohl mit als auch ohne Zusatz von Heparin-neutralisierenden Agentien zur Bestimmung der aPTT eingesetzt.
Diese Reagentien weisen folgende Zusammensetzung auf (die % sind Gew.%):
- Reagens A:: 0.075 % Kaolin und 0.01 % Sulfatid (Fa. Pentapharm) als Initiator, hochgereinigte Phospholipide (17 % Phosphatidylserin, 44 % Phosphatidylcholin, 29 % Phosphatidylethanolamin, 5 % Cholesterin und 5 % Phosphatidinsäure).
Das gebrauchsfertige Reagens wird durch Rekonstitution mit 2 ml Aqua dest. hergestellt (Konzentration der Phospholipide: 65 µg/ml).
- Reagens B:: wie Reagens A, jedoch nur 0.0017 % Sulfatid
- Reagens C:: 0.5 % Kaolin, Lipidextrakt aus Schweinemucosa (Tachostyptan®, Fa. Hormonchemie).
Das gebrauchsfertige Reagens wird durch Rekonstitution mit 2 ml Aqua dest. hergestellt.
- Reagens D:: Aktin FS® der Fa. Baxter (enthält Ellagsäure als Initiator und Phospholipide aus Sojabohne).
- Reagens E:: Pathromtim® der Fa. Behring (enthält 0.5 % Kaolin in flüssiger Suspension als Initiator und Lipidextrakt aus humaner Plazenta (lyo)).
Das gebrauchsfertige Reagens wird durch Lösen des Lipidextraktes in der Kaolinsuspension hergestellt.

Ein Normalplasma (NP; Immunocontrolplasma Normal der Fa. Immuno) bzw. heparinisiertes Normalplasma mit 0.4 E Heparin/ml (HP1) oder 1.0 E Heparin/ml (HP2) wurden als Plasmaproben verwendet. 0.1 ml Plasmaprobe wurden mit 0.1 ml Reagens versetzt. Nach einer zweiminütigen Inkubationszeit bei 37°C wurden 0.1 ml 0.025 M CaCl₂-Lösung zugesetzt und die Zeit bis zum Gerinnungspunkt mit einem Schnitger-Gross-Koagulometer (Fa. Amelung) gemessen.

Tabelle 6 zeigt die erhaltenen Resultate, wobei in Zeile 1 die Gerinnungszeit (in Sekunden) von Normalplasma (NP), in den Zeilen 2 und 3 die Gerinnungszeit der heparinisierten Plasmen (HP1 und HP2) und in den Zeilen 4 und 5 das Verhältnis zwischen den Gerinnungszeiten der heparinisierten Plasmen und von Normalplasma angegeben wird.

**Tabelle 6**

| | Reagenz A | | Reagenz C | | Reagenz D | | Reagenz E | |
|---|---|---|---|---|---|---|---|---|
| | o Polybren | 25mg/l P. | o. Polybren | 25mg/l P. | o. Polybren | 25mg/l P. | o. Polybren | 50mg/IP. |
| NP sec. | 34.0 | 32.4 | 52.4 | 68.0 | 43.1 | 65.9 | 34.1 | 46.6 |
| HP1 sec. | 52.9 | 36.1 | 123.1 | 75.8 | 99.8 | 67.6 | 76.1 | 50.9 |
| HP2 sec. | 214.1 | 39.1 | 253.4 | 80.3 | 283.4 | 59.1 | 210.1 | 96.2 |
| | | | | | | | | |
| Ratio HP1/NP | 1.56 | 1.11 | 2.35 | 1.11 | 2.32 | 1.03 | 2.23 | 1.09 |
| Ratio HP2/NP | 6.30 | 1.21 | 4.84 | 1.18 | 6.58 | 0.90 | 6.16 | 2.06 |
| o. Polybren = ohne Polybren P. = Polybren | | | | | | | | |

Die Resultate zeigen deutlich, daß die aPTT bei heparinisierten Plasmen ohne Anwesenheit von Polybren deutlich verlängert und daher verfälscht bzw. verzerrt wird. Bei Anwesenheit von Polybren kann mit allen getesteten Reagentien die aPTT auch von heparinisierten Plasmen bestimmt werden. Das Verhältnis zwischen den Gerinnungszeiten der heparinisierten Plasmen und von Normalplasma, welches im Idealfall gleich 1 ist (d.h. die Gerinnungszeit ist exakt dieselbe), weicht bei den Tests mit den Reagentien ohne Polybren ganz erheblich vom Idealwert 1 ab, wohingegen das Verhältnis der Gerinnungszeiten bei Anwesenheit von Polybren nahe bei 1 liegt.

### 6. Faktor VIII-Bezugskurve mit PTT Reagentien unterschiedlicher Zusammensetzungen mit und ohne Zusatz von Polybren.

Zur Erstellung dieser Bezugskurven wurde ein Referenzplasma (Reference Plasma 100 % der Fa. Immuno) verwendet. Dieses Referenzplasma wurde in 1 ml Aqua dest. (RP) bzw. mit 1 ml Aqua dest., enthaltend 1 E/ml Heparin (Fa. Immuno) (HP), gelöst.

Die Plasmen wurden 1:5 mit Imidazolpuffer (3.4 g/l Imidazol, 5.85 g/l NaCl, pH 7.4) vorverdünnt; dies entspricht 100 % Faktor VIII. Weitere Verdünnungen wurden gemäß einer geometrischen Verdünnungsreihe (1:1 = 100 % bis 1:128 = 0.78 %) hergestellt. 0.1 ml der Probe wurden mit 0.1 ml Reagens vermischt und 4 min bei 37°C inkubiert. Anschließend wurden 0.1 ml 0.025 M CaCl₂-Lösung zugesetzt und die Zeit bis zum Gerinnungspunkt mit einem Schnitger-Gross-Koagulometer gemessen.

Die Ergebnisse sind in den Tabellen 7 bis 10 dargestellt.

**Tabelle 7**

| Reagenz A | | | | |
|---|---|---|---|---|
| | o. Polybren | | 25 mg/ml Polybren | |
| F.VIII (%) | RP | HP | RP | HP |
| 100 | 54.7 | 82.0 | 40.0 | 42.2 |
| 50 | 65.2 | 78.6 | 48.6 | 47.6 |
| 25 | 73.5 | 82.1 | 52.0 | 52.1 |
| 12.5 | 81.7 | 86.6 | 58.3 | 58.0 |
| 6.25 | 90.7 | 92.6 | 64.5 | 64.6 |
| 3.13 | 98.1 | 104.1 | 70.6 | 71.6 |
| 1.56 | 107.2 | 108.6 | 78.6 | 78.6 |
| 0.78 | 115.1 | 117.6 | 83.6 | 83.6 |

**Tabelle 8**

| Reagenz C | | | | |
|---|---|---|---|---|
| | o. Polybren | | 25 mg/ml Polybren | |
| F.VIII (%) | RP | HP | RP | HP |
| 100 | 81.6 | 129.1 | 90.1 | 87.6 |
| 50 | 91.3 | 105.6 | 99.6 | 99.6 |
| 25 | 104.5 | 102.1 | 113.1 | 110.6 |
| 12.5 | 110.6 | 109.1 | 123.1 | 127.6 |
| 6.25 | 121.5 | 120.1 | 138.1 | 134.6 |
| 3.13 | 130.5 | 132.1 | 150.1 | 151.1 |
| 1.56 | 141.6 | 143.1 | 163.1 | 163.1 |
| 0.78 | 150.5 | 151.6 | 177.5 | 175.1 |

**Tabelle 9**

| Reagenz D | | | | |
|---|---|---|---|---|
| | o. Polybren | | 25 mg/ml Polybren | |
| F.VIII (%) | RP | HP | RP | HP |
| 100 | 60.6 | 107.2 | 70.1 | 65.6 |
| 50 | 71.7 | 84.1 | | |
| 25 | 79.0 | 82.6 | 100.1 | 93.6 |
| 12.5 | 88.1 | 89.6 | | |
| 6.25 | 98.1 | 100.1 | | |
| 3.13 | 109.1 | 109.6 | 140.6 | 138.6 |
| 1.56 | 118.1 | 118.2 | | |
| 0.78 | 127 | 126.6 | 164 | 160.5 |

**Tabelle 10**

| Reagenz E | | | | |
|---|---|---|---|---|
| | o. Polybren | | 25 mg/ml Polybren | |
| F.VIII (%) | RP | HP | RP | HP |
| 100 | 54.9 | 127.5 | 72.1 | 68.1 |
| 50 | 61.2 | 98.5 | 78.4 | 75.1 |
| 25 | 67.4 | 96.4 | 87.9 | 82.6 |
| 12.5 | 74.2 | 102.4 | 94.9 | 92.1 |
| 6.25 | 83.4 | 112.6 | 106.2 | 105.1 |
| 3.13 | 89.9 | 120.6 | 113.2 | 115.6 |
| 1.56 | 97.1 | 131.6 | 121.1 | 125.5 |
| 0.78 | 102.4 | 140.9 | 127.6 | 131.6 |

Die Ergebnisse zeigen, daß im Normalfall (Plasma ohne Heparin; RP, Reagentien ohne Polybren) eine eindeutige Korrelation zwischen der Gerinnungszeit und der Faktor VIII-Konzentration besteht: Je geringer die Konzentration an Faktor VIII, desto länger die Gerinnungszeit.

Bei der Messung von Heparin-hältigen Proben (HP, Reagentien o. Polybren) zeigt sich keine eindeutige Korrelation, die Messung ergibt eine unbrauchbare Faktor VIII-Bezugskurve, die keine lineare Zuordnung von Gerinnungszeit und Faktor VIII-Konzentration ermöglicht.

Erst die Anwesenheit von Polybren in den Reagentien ermöglicht eine Bestimmung der Faktor VIII-Konzentration in heparinhältigen Proben (HP, Reagentien mit 12.5/25/50 mg/ml Polybren). Die Ergebnisse der Messungen werden durch die Anwesenheit von Heparin aber auch Polybren bei Verwendung des erfindungsgemäßen Reagens nicht verfälscht. Dies zeigt sich sowohl beim Vergleich der Messungen von RP mit Reagentien mit und ohne Polybren, als auch beim Vergleich der Gerinnungszeiten von RP und HP in Anwesenheit von Polybren, deren Quozient immer ganz nahe bei 1 liegt.

### 7. aPTT-Bestimmung mit Reagens B mit und ohne Zusatz von Heparinase.

Immunocontrolplasma Normal (Fa. Immuno) wurde in 1 ml Aqua dest. gelöst, das 0, 0.5 oder 1 U/ml Heparin enthielt.

Reagens B wurde in 1 ml Aqua dest. gelöst, welches 0, 0.74, 0.22, 0.07 und 0.02 E/ml Heparinase (Fa. IBEX, Kanada) enthielt.

Die PTT-Bestimmung wurde wie in Beispiel 5 beschrieben, durchgeführt.

Die Ergebnisse der durchgeführten Messungen sind in Tabelle 11 dargestellt.

**Tabelle 11**

| heparin in plasma U/ml | heparinase U/ml | | | | |
|---|---|---|---|---|---|
| | 0 | 0.02 | 0.07 | 0.22 | 0.74 |
| 0.0 | 33.6 | 33.6 | 33.1 | 33.6 | 38.3 |
| 0.5 | 52.4 | 45.8 | 41.8 | 37.7 | 41.4 |
| 1.0 | 85.8 | 65.6 | 50.8 | 47.3 | 42.3 |

Diese Ergebnisse zeigen, daß sich auch ein Reagens enthaltend Heparinase vorzüglich zur aPTT-Bestimmung von heparinisiertem Plasma eignet.

## Patentansprüche

1. Initiator der intrinsischen Gerinnung zur Bestimmung der Gerinnungszeit von Blutplasma oder Blutderivaten, dadurch gekennzeichnet, daß er ein Gemisch aus Sulfatiden und einem Feststoffaktivator, vorzugsweise Kaolin, enthält.

2. Reagens zur Bestimmung der aPTT von Blut, Plasma oder Blutderivaten, dadurch gekennzeichnet, daß es ein Gemisch aus Sulfatiden und einem Feststoff-Aktivator, vorzugsweise Kaolin, als Initiator der intrinsischen Gerinnung und weiters Phospholipide enthält.

3. Reagens nach Anspruch 2, dadurch gekennzeichnet, daß die enthaltenen Phospholipide chemisch reine Phospholipide in einer standardisierten Mischung sind.

4. Reagens nach Anspruch 3, dadurch gekennzeichnet, daß die Phospholipidmischung, bezogen auf den Gesamtphospholipidgehalt, 5 bis 50 % Phosphatidylserin, 15 bis 60 % % Phosphatidylcholin, 15 bis 60 % Phosphatidylethanolamin, 0 bis 20 % Cholesterin und 5 bis 20 % Phosphatidinsäure enthält.

5. Reagens nach einem oder mehreren der Ansprüche 2 bis 4, dadurch gekennzeichnet, daß es weiters Oxidationshemmer und/oder Empfindlichkeitsverbesserer enthält.

6. Reagens nach Anspruch 5, dadurch gekennzeichnet, daß es Alkalisalze, eventuell gemeinsam mit Aminosäuren, enthält.

7. Reagens nach einem oder mehreren der Ansprüche 2 bis 6, dadurch gekennzeichnet, daß es weiters ein chromogenes Substrat enthält, mit dem gegebenenfalls die Gerinnungszeit photometrisch durch Farbentwicklung bestimmbar ist.

8. Reagens nach einem oder mehreren der Ansprüche 2 bis 7, dadurch gekennzeichnet, daß es weiters Stabilisatoren, vorzugsweise Albumin und/oder Puffersubstanzen enthält.

9. Reagens nach einem oder mehreren der Ansprüche 2 bis 8, dadurch gekennzeichnet, daß es in lyophilisierter Form vorliegt.

10. Verfahren zur Herstellung des Reagens nach einem oder mehreren der Ansprüche 2 bis 9, dadurch gekennzeichnet, daß Sulfatide in einer wässerigen Suspension des Feststoff-Aktivators mit Phospholipiden und gegebenenfalls mit weiteren Zusätzen vermischt werden und gegebenenfalls lyophilisiert werden.

11. Verfahren zur Herstellung des Reagens nach einem oder mehreren der Ansprüche 2 bis 9, dadurch gekennzeichnet, daß Sulfatide, der Feststoffaktivator und Phospolipide, gegebenenfalls nach Lyophilisierung, gegebenenfalls mit weiteren Zusätzen im festen Zustand vermischt werden.

12. Verfahren zur Bestimmung der partiellen Thromboplastinzeit (aPTT) von Proben von Blut, Plasma oder Blutderivaten, dadurch gekennzeichnet, daß die Probe mit einem Reagens nach einem oder mehreren der Ansprüche 2 bis 9 in Kontakt gebracht wird, nach einer vorbestimmten Aktivierungszeit Calciumionen zugesetzt werden und die Gerinnungszeit bestimmt wird.

13. Verfahren nach Anspruch 12, dadurch gekennzeichnet, daß die Probe vor Zugabe eines Reagens nach einem der Ansprüche 2 bis 9 mit einer Heparin-neutralisierenden Substanz versetzt wird.

14. Verwendung des Verfahrens nach Anspruch 12 oder 13 zur Bestimmung der Aktivität von Faktoren der intrinsischen Gerinnung und deren Inhibitoren, dadurch gekennzeichnet, daß eine Plasmaprobe mit einem Reagens nach den Ansprüchen 2 bis 9 in Kontakt gebracht wird, nach einer vorbestimmten Aktivierungszeit Calciumionen zugesetzt werden und die Gerinnungszeit bestimmt wird, worauf das erhaltene Ergebnis mit dem einer Standardprobe mit bekannter Aktivität der Faktoren in Bezug gesetzt wird.

15. Reagens nach einem der Ansprüche 2 bis 9 zur Bestimmung der aPTT von gegebenenfalls Heparin- oder Heparinoid-hältigem(n) Blut, Plasma oder Blutderivaten, dadurch gekennzeichnet, daß es ein Heparin-neutralisierendes Agens enthält.

16. Reagens nach Anspruch 15, dadurch gekennzeichnet, daß es Polybren enthält.

17. Reagens nach Anspruch 15 oder 16, dadurch gekennzeichnet, daß es ein Protaminsalz oder Heparinase enthält.

18. Reagens nach einem der Ansprüche 15 bis 17, dadurch gekennzeichnet, daß die Phospholipidkomponente, bezogen auf den Gesamtphospholipidgehalt, 5 bis 50 % Phosphatidylserin, 15 bis 60 % Phosphatidylcholin, 15 bis 60 % Phosphatidylethanolamin, 0 bis 20 % Cholesterin und 5 bis 20 % Phosphatidinsäure enthält.

19. Verfahren zur Bestimmung der partiellen Thromboplastinzeit (aPTT) von Proben von gegebenenfalls Heparin- oder Heparinoidhältigem(n) Blut, Plasma oder Blutderivaten, dadurch gekennzeichnet, daß die Probe mit einem Reagens nach einem oder mehreren der Ansprüche 15 bis 18 in Kontakt gebracht wird, nach einer vorbestimmten Aktivierungszeit Calciumionen zugesetzt werden und die Gerinnungszeit bestimmt wird.

20. Verwendung des Verfahrens nach Anspruch 19 zur Bestimmung der Aktivität von Faktoren der intrinsischen Gerinnung und deren Inhibitoren, dadurch gekennzeichnet, daß eine gegebenenfalls Heparin- oder Heparinoid-hältige Plasmaprobe mit einem Reagens nach den Ansprüchen 15 bis 18 in Kontakt gebracht wird, nach einer vorbestimmten Aktivierungszeit Calciumionen zugesetzt werden und die Gerinnungszeit bestimmt wird, worauf das erhaltene Ergebnis mit einer Standardprobe mit bekannter Aktivität der Faktoren in Bezug gesetzt wird.

21. Set, bestehend aus
a) einem Reagens zur Bestimmung der aPTT nach einem der Ansprüche 2 bis 9, und
b) einem Heparin-neutralisierenden Agens.

## Claims

1. An initiator of intrinsic coagulation for determining the coagulation time of blood plasma or blood derivatives, characterised in that it comprises a mixture of sulfatides and a solid activator, preferably kaolin.

2. A reagent for determining the aPTT of blood, plasma or blood derivatives, characterised in that it comprises a mixture of sulfatides and a solid activator, preferably kaolin, as initiator of the intrinsic coagulation, and furthermore, phospholipids.

3. A reagent according to claim 2, characterised in that the phospholipids comprised are chemically pure phospholipids in a standardized mixture.

4. A reagent according to claim 3, characterised in that the phospholipid mixture comprises from 5 to 50 % phosphatidyl serine, from 15 to 60 % phosphatidyl choline, from 15 to 60 % phosphatidyl ethanol amine, from 0 to 20 % cholesterol and from 5 to 20 % phosphatidinic acid, based on the total phospholipid content.

5. A reagent according to any one or several of claims 2 to 4, characterised in that it further comprises oxidation inhibitors and/or sensitivity promoters.

6. A reagent according to claim 5, characterised in that it comprises alkali salts, possibly together with amino acids.

7. A reagent according to any one or several of claims 2 to 6, characterised in that it further comprises a chromogenic substrate, by which, if desired, the coagulation time can be determined photometrically by colour development.

8. A reagent according to any one or several of claims 2 to 7, characterised in that it further comprises stabilizers, preferably albumin, and/or buffer substances.

9. A reagent according to any one or several of claims 2 to 8, characterised in that it is present in lyophilized form.

10. A method of preparing a reagent according to any one or several of claims 2 to 9, characterised in that sulfatides are mixed in an aqeuous suspension of the solid activator with phospholipids, and, optionally, with further additives, and, optionally, are lyophilised.

11. A method of preparing the reagent according to any one or several of claims 2 to 9, characterised in that sulfatides, the solid activator and phospholipids, are mixed, optionally after lyophilizing, optionally with further additives, in the solid state.

12. A method of determining the partial thromboplastin time (aPTT) of samples of blood, plasma or blood derivatives, characterised in that the sample is contacted with a reagent according to any one or several of claims 2 to 9, calcium ions are added after a pre-determined activation time, and the coagulation time is determined.

13. A method according to claim 12, characterised in that the sample is admixed with a heparin-neutralizing substance before the reagent according to any one of claims 2 to 9 is added.

14. The use of the method according to claim 12 or 13 for determining the activity of factors of intrinsic coagulation and the inhibitors thereof, characterised in that a plasma sample is contacted with a reagent according to claims 2 to 9, calcium ions are added after a pre-determined activation time, and the coagulation time is determined, whereupon the result obtained is correlated to a standard sample with a known activity of the factors.

15. A reagent according to any one of claims 2 to 9 for determining the aPTT of possibly heparin- or heparinoid-containing blood, plasma or blood derivatives, characterised in that it comprises a heparin-neutralizing agent.

16. A reagent according to calim 15, characterised in that it comprises polybren.

17. A reagent according to claim 15 or 16, characterised in that it comprises a protamin salt or heparinase.

18. A reagent according to any one of claims 15 to 17, characterised in that the phospholipid component comprises 5 to 50 % of phosphatidyl serine, 15 to 60 % of phosphatidyl choline, 15 to 60 % of phosphatidyl ethanol amine, 0 to 20 % of cholesterol and 5 to 20 % of phosphatidinic acid, based on the total phospholipid content.

19. A method of determining the partial thromboplastin time (aPTT) of samples of possibly heparin- or heparinoid-containing blood, plasma or blood derivatives, characterised in that the sample is contacted with a reagent according to any one or several of claims 15 to 18, calcium ions are added after a pre-determined activation time, and the coagulation time is determined.

20. The use of a method according to claim 19 for determining the activity of factors of intrinsic coagulation in the inhibitors thereof, characterised in that a possibly heparin- or heparinoid-containing plasma sample is contacted with a reagent according to claims 15 to 18, calcium ions are added after a pre-determined activation time, and the coagulation time is determined, whereupon the result obtained is correlated to the result of a standard sample of known activity of the factors.

21. A set, comprised of
a) a reagent for determining the aPTT according to any one of claims 2 to 9, and
b) a heparin-neutralizing agent.

## Revendications

1. Initiateur de coagulation intrinsèque pour la détermination du temps de coagulation de plasma sanguin ou de dérivés sanguins, caractérisé en en ce qu'il contient un mélange de sulfatides et d'un facteur solide, de préférence du kaolin.

2. Réactif pour la définition de l'aPTT du sang, du plasma ou de dérivés sanguins, caractérisé en ce qu'il contient un mélange de sulfatides et d'un activateur solide, de préférence du kaolin, comme initiateur de la coagulation intrinsèque et en outre des phospholipides.

3. Réactif selon la revendication 2, caractérisé en ce que les phospholipides contenus sont des phopsholipides purs chimiquement dans un mélange standardisé.

4. Réactif selon la revendication 3, caractérisé en ce que le mélange de phospholipides, par rapport à la teneur totale des phospholipides, contient de 5 à 50 % de phosphatidylsérine, 15 à 60 % de phosphatidylcholine, 15 à 60 % de phosphatidyléthanolamine, 0 à 20 % de cholestérine et 5 à 20 % d'acide phosphatidinique.

5. Réactif selon l'une ou plusieurs des revendications 2 à 4, caractérisé en ce qu'il contient en outre un inhibiteur d'oxydation et/ou un agent améliorant la sensibilité.

6. Réactif selon la revendication 5, caractérisé en ce qu'il contient des sels alcalins, éventuellement simultanément avec de acides aminés.

7. Réactif selon l'une ou plusieurs des revendications 2 à 6, caractérisé en ce qu'il contient en outre un substrat chromogène, avec lequel éventuellement le temps de coagulation est définissable photométriquement par développement de couleur.

8. Réactif selon l'une ou plusieurs des revendications 2 à 7, caractérisé en ce qu'il contient en outre des stabilisants, de préférence de l'albumine et/ou des substances tampons.

9. Réactif selon l'une ou plusieurs des revendications 2 à 8, caractérisé en ce qu'il est sous forme lyophilisée.

10. Procédé de préparation d'un réactif selon l'une ou plusieurs des revendications 2 à 9, caractérisé en ce qu'on mélange les sulfatides dans une suspension aqueuse de l'activateur solide avec des phospholipides et éventuellement d'autres additifs et éventuellement on les lyophilise.

11. Procédé de préparation d'un réactif selon l'une ou plusieurs des revendications 2 à 9, caractérisé en ce qu'on mélange les sulfatides, l'activateur solide et les phospholipides, éventuellement après lyophilisation, éventuellement avec d'autres additifs à l'état solide.

12. Procédé pour la détermination du temps de thromboplastine partiellement activée (aPTT) d'échantillons de sang, de plasma ou de dérivés sanguins, caractérisé en ce qu'on met en contact les échantillons avec un réactif selon l'une ou plusieurs des revendications 2 à 9, on ajoute des ions calcium après un temps d'activation prédéterminé et on détermine le temps de coagulation.

13. Procédé selon la revendication 12, caractérisé en ce que l'échantillon avant l'addition d'un réactif selon l'une des revendications 2 à 9, est additionné d'une substance neutralisant l'héparine.

14. Utilisation du procédé selon les revendications 12 ou 13 pour la définition de l'activité des facteurs de la coagulation intrinsèque et de leurs inhibiteurs, caractérisée en ce qu'on met en contact un échantillon de plasma avec un réactif selon les revendications 2 à 9, on leur ajoute des ions calcium après un temps d'activation prédéterminé et on détermine le temps de coagulation, le résultat obtenu avec l'un des échantillons standards étant rapporté à l'activité connue des facteurs.

15. Réactif selon l'une des revendications 2 à 9 pour la détermination de l'aPTT de sang, de plasma ou de dérivés sanguins contenant éventuellement de l'héparine ou de l'héparinoïde, caractérisé en ce qu'il contient un agent de neutralisation de l'héparine.

16. Réactif selon la revendication 15, caractérisé en ce qu'il contient du polybrène.

17. Réactif selon la revendication 15 ou 16, caractérisé en ce qu'il contient un sel de protamine ou de l'héparinase.

18. Réactif selon l'une des revendications 15 à 17, caractérisé en ce que les composants de phospholipides contiennent, par rapport à la teneur totale en phospholipides, 5 à 50 % de phosphatidylsérine, 15 à 60 % de phosphatidylcholine, 15 à 60 % de phosphatidyléthanolamine, 0 à 20 % de cholestérine et 5 à 20 % d'acide phosphatidinique.

19. Procédé pour la détermination du temps de thromboplastine partiellement activée (aPTT) d'échantillons de sang, de plasma ou de dérivés sanguins contenant éventuellement de l'héparine ou de l'héparinoïde, caractérisé en ce qu'on met en contact l'échantillon avec un réactif selon l'une ou plusieurs des revendications 15 à 18, on ajoute des ions calcium après un temps d'activation prédéterminé et on détermine le temps de coagulation.

20. Utilisation du procédé selon la revendication 19 pour la détermination de l'activité de facteurs de la coagulation intrinsèque et de leurs inhibiteurs, caractérisé en ce qu'on amène en contact un échantillon de plasma contenant éventuellement de l'héparine ou de l'héparinoïde avec un réactif selon les revendications 15 à 18, on ajoute des ions calcium après un temps d'activation prédéterminé et on détermine le temps de coagulation, le résultat obtenu avec un des échantillons standards étant rapporté à l'activité connue des facteurs.

21. Ensemble constitué de
a) un réactif pour la définition de l'aPTT selon l'une des revendications 2 à 9, et
b) un agent neutralisant l'héparine.
